# FASCICULE DE BREVET EUROPEEN

(11) **EP 0 971 754 B1**
(45) Date de publication et mention de la délivrance du brevet: **27.06.2001**
(21) Numéro de dépôt: 98913459.8
(22) Date de dépôt: 02.04.1998
(51) Int. Cl.: A61M 1/00

(54) **APPAREIL DE LIPO-ASPIRATION**
FETTSAUGVORRICHTUNG
LIPOSUCTION APPARATUS

(30) Priorité: 03.04.1997 BE 9700305
(43) Date de publication de la demande: 19.01.2000
(73) Titulaire: Malak, Jean Clément Edouard Ghislain, B-7130 Binche (BE)
(72) Inventeur: Malak, Jean Clément Edouard Ghislain, B-7130 Binche (BE)
(74) Mandataire: Quintelier, Claude
(86) Numéro de dépôt international: BE9800047
(87) Numéro de publication internationale: WO9844966

(56) Documents cités:
- WO-A-93/02627
- DE-A- 3 313 325
- FR-A- 2 559 066
- FR-A- 2 648 050
- FR-A- 2 691 624
- US-A- 4 735 604
- US-A- 4 863 439
- US-A- 5 348 535
- US-A- 5 352 194

## Description

La présente invention concerne un appareil de lipo-aspiration comprenant une canule d'aspiration agencée pour aspirer de la graisse sous-cutanée par un orifice d'entrée, la canule ayant un axe longitudinal et étant montée sur un organe d'entraînement mécanique ayant une entrée pourvue pour y connecter une source d'énergie et agencé pour produire un mouvement et le transmettre à ladite canule et le mouvement de la canule comprenant un composant de translation suivant l'axe de la canule.

La technique même de la lipo-aspiration est connue en tant que telle. La canule d'aspiration sert à être introduite sous la peau du patient. La canule est enfoncée jusqu'à la zone où la graisse doit être aspirée. L'aspiration même de la graisse est accompagnée d'un mouvement de va et vient répété et d'une dépression créée à l'intérieur de la canule.

Pour assister l'utilisateur lors de la lipo-aspiration, des appareils à assistance mécanique ont été conçus munis d'un organe d'entraînement mécanique pour produire un mouvement et le transmettre à la canule. Dans ces appareils, comme par exemple décrit dans le brevet US 5,348,535, le mouvement produit est un mouvement de translation appliqué à un organe de raclage. Afin de guider le mouvement de translation de cet organe, ces appareils comprennent une canule intérieure qui coulisse dans une canule extérieure de guidage. La canule extérieure sert donc à guider le mouvement de translation de la canule intérieure, tandis que la canule intérieure sert de canule d'aspiration.

L'inconvénient de l'appareil suivant US 5,348,535 est que le mouvement de translation de la canule intérieure par rapport à la canule extérieure entraîne un effet de "guillotine" ou de cisaillement, c'est-à-dire qu'il peut provoquer une coupure des vaisseaux et des nerfs. Cet effet de guillotine impose pratiquement impérativement une anesthésie générale du patient. De plus, la graisse risque de s'infiltrer entre la canule intérieure et la canule extérieure, ce qui peut provoquer un blocage du mouvement de translation. L'appareil connu présente également l'inconvénient que, eu égard à la présence d'une double canule, c'est-à-dire une canule intérieure coulissant dans une canule extérieure, le diamètre de la canule est relativement élevé. Si l'on souhaite diminuer le diamètre de la canule extérieure pour pouvoir accéder plus facilement dans le corps du patient, le diamètre de la canule intérieure sera également réduit, ce qui réduit le débit d'aspiration de graisse.

Un autre appareil de lipo-aspiration est décrit dans le brevet US 5,352,194. L'organe d'entraînement de cet appareil est agencé à produire un mouvement de translation ayant une amplitude d'au moins 1 cm. Une telle amplitude produit un effet de raclement qui est dangereux car l'opérateur risque de dépasser les limites de la région à liposucer et ainsi de blesser le patient. En outre, plus l'amplitude est grande, plus la réaction est forte, ce qui fait reculer sans arrêt le poignet de l'opérateur et diminue ainsi la précision du travail accompli.

Un autre appareil de lipo-aspiration est décrit dans la demande de brevet FR 2 691 624. Cet appareil est un appareil à ultrasons, dans lequel l'organe d'entraînement est agencé à produire un mouvement de vibration à fréquence ultrason. Un tel appareil s'est avéré peu efficace, étant donné que le mouvement de vibration à fréquence ultrason doit faire éclater les cellules de graisse pour les aspirer ensuite. Cette technique est très lente. De plus de graves brûlures et des nécroses cutanées peuvent se produire.

Le document US 4,735,604 décrit un appareil d'extraction de matière biologique. Le tube est maintenu droit par un manche et une pièce extrême. Le mouvement produit est limité à un mouvement de translation inférieure ou égale à 1 mm. Avec ce type d'appareil, il n'est pas possible d'effectuer une lipo-aspiration de façon efficace.

La présente invention a pour but de réaliser un appareil de lipo-aspiration dont l'usage entraîne considérablement moins de lésions des vaisseaux et des nerfs tout en apportant une assistance mécanique qui permet de prélever efficacement la graisse sous-cutanée du patient.

L'appareil de lipo-aspiration suivant l'invention comprend une canule d'aspiration agencée pour aspirer de la graisse sous-cutanée par un orifice d'entrée, la canule ayant un axe longitudinal; un organe d'entraînement mécanique sur lequel est monté la canule, l'organe d'entraînement ayant une entrée pourvue pour y connecter une source d'énergie et agencé pour produire un mouvement et le transmettre à ladite canule; et un boîtier à l'intérieur duquel l'organe d'entraînement est logé. Le mouvement de la canule est un mouvement de nutation comprenant un composant de vibration perpendiculaire à l'axe de la canule et un composant de translation suivant l'axe de la canule, le composant de translation ayant une amplitude entre 2 mm et 1 cm. Un espace libre est prévu entre la canule et le boîtier, ledit espace étant suffisamment dimensionné de façon à permettre au composant de vibration de provoquer la dislocation de la graisse.

La transmission d'un tel mouvement de nutation à la canule a un double effet. D'une part, le composant de vibration permet à la canule d'effectuer un mouvement de vibration quand cette dernière est située dans le tissu graisseux sous-cutané. Ce mouvement va induire une vibration dans la graisse, permettant de facilement faire entrer la graisse dans la canule. D'autre part, le composant de translation amplifie celui de vibration et assure ainsi une lipo-aspiration efficace. Le mouvement de translation permet également de faire progresser très facilement et avec plus de douceur la canule sous la peu du patient. En limitant l'amplitude du composant de translation à une valeur inférieure à 1 cm, la graisse peut facilement être aspirée jusqu'à sa limite d'extension tout en limitant considérablement le risque de dépasser la zone à traiter. Une valeur minimale de 2 mm est choisie de préférence. Le mouvement transmis par l'organe d'entraînement crée une onde sinusoïdale qui se prolonge le long de la canule entraînant une force de nutation qui contribue à disloquer la graisse et à réaliser une véritable émulsion du tissu adipeux.

On a constaté lors d'essais, que peu de sang était extrait par la canule, ce qui indique clairement que la lipo-aspiration effectuée avec l'appareil selon l'invention limite considérablement les lésions des vaisseaux et des nerfs par rapport aux appareils à assistance mécanique connus, dans lesquels il y a soit un effet de guillotine, soit un effet de raclement, soit une lipo-aspiration inefficace. Une anesthésie locale est donc suffisante. Par l'absence de deux canules coulissant l'une dans l'autre, le diamètre de la canule introduit sous la peau du patient peut être relativement limité, ce qui permet de plus facilement accéder à des zones du corps humain ayant un accès relativement restreint, tels que le menton et la cheville, et limite en plus la grandeur des cicatrices formées sur la peau du patient.

Dans une première forme de réalisation préférentielle d'un appareil suivant l'invention, ledit mouvement produit par l'organe d'entraînement a une fréquence entre 10 et 500 Hz, en particulier inférieure à 250 Hz, et plus particulièrement environ égale à 15 Hz. Il s'est avéré qu'une telle plage de fréquence permet d'obtenir des résultats efficaces en combinaison avec le mouvement de translation d'une amplitude inférieure à 1 cm.

Dans une deuxième forme de réalisation préférentielle, l'appareil suivant l'invention comporte une poignée dans laquelle ledit organe d'entraînement est logé. Ceci facilite la prise en main de l'appareil suivant l'invention.

De préférence, l'appareil comporte en outre un organe de commande agencé à commander la mise en marche et l'arrêt de l'organe d'entraînement. En particulier, l'organe de commande est un interrupteur, logé dans ladite poignée, ou est formé par une pédale de commande coopérant avec ledit organe d'entraînement. L'opérateur peut ainsi facilement commander la mise en marche et l'arrêt de l'appareil, en particulier en commandant et tenant l'appareil par la même main ou en commandant l'appareil à l'aide d'un de ses pieds.

De préférence ledit organe d'entraînement est agencé pour fonctionner à l'air comprimé et comporte un piston pneumatique pour produire un mouvement de va-et-vient. L'utilisation d'air comprimé permet de réaliser un appareil qui est facile à stériliser.

Dans une forme de réalisation préférentielle de l'appareil de lipo-aspiration selon l'invention, l'appareil comporte en outre une cavité pour un pouce, ladite cavité et ledit orifice étant positionnés de part et d'autre de l'axe de la canule. Cette forme de réalisation provoque que, lorsque l'opérateur, qui a introduit la canule sous la peau d'un patient, place son pouce dans la cavité, l'orifice d'entrée soit dirigé vers la profondeur du tissu adipeux et non vers la peau du patient.

L'invention sera maintenant décrite plus en détail à l'aide des dessins qui illustrent un exemple de réalisation d'un appareil suivant l'invention. Dans les dessins:
La figure 1 illustre une vue d'ensemble d'un appareil de lipo-aspiration suivant l'invention;

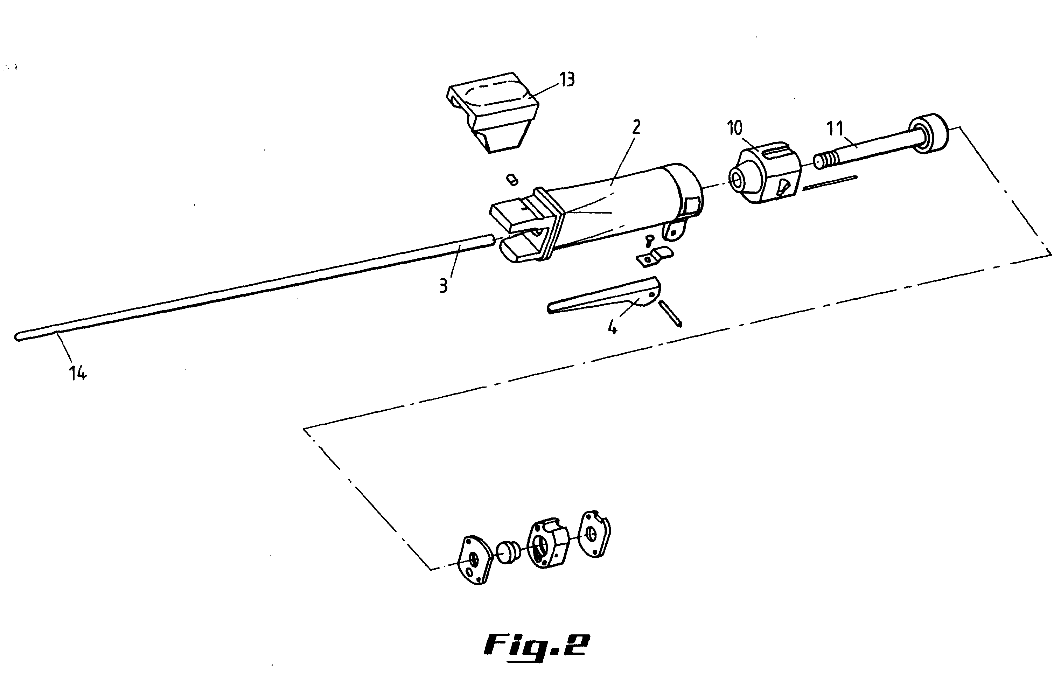

La figure 2 illustre les composants principaux d'un appareil suivant l'invention.
La figure 3 illustre une partie d'une forme de réalisation préférentielle de l'appareil suivant l'invention.
La figure 4 est une vue en coupe d'une partie de la figure 3.
La figure 5 illustre schématiquement le mouvement de nutation transmis à la canule.
Les figures 6 à 8 montrent une vue en coupe des différentes phases du fonctionnement d'un moteur permettant de produire le mouvement à imposer à la canule.
Les figures 9 a à e illustrent la configuration des circuits d'admission et d'échappement du moteur.

Dans les dessins une même référence a été attribuée à un même élément ou à un élément analogue.

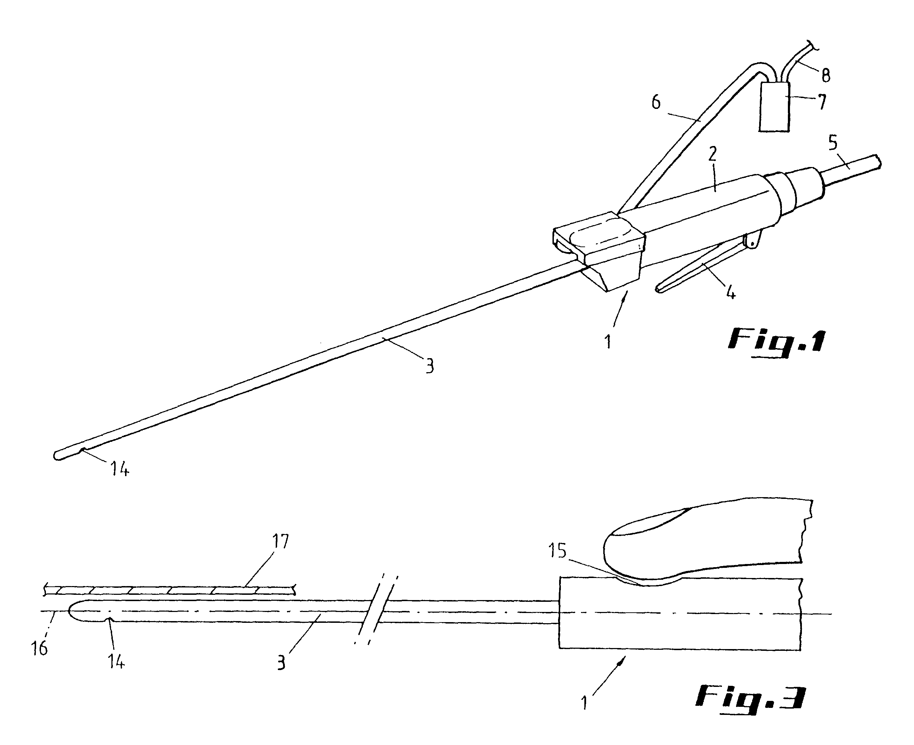

La figure 1 montre un exemple de réalisation d'un appareil de lipo-aspiration 1 suivant l'invention. Dans l'exemple repris l'appareil utilise du gaz comprimé, de préférence de l'air comprimé, en particulier de l'air médical stérile déshydraté, comme source d'énergie. Eu égard à l'usage médical l'appareil à air comprimé a l'avantage d'être facilement stérilisé. Toutefois d'autres sources d'énergie peuvent être utilisées comme par exemple du courant électrique, de l'induction magnétique ou un liquide sous pression. L'avantage toutefois d'utiliser de l'air comprimé par rapport au courant électrique est qu'il évite sensiblement de surchauffer l'appareil lorsque le mouvement est bloqué, par exemple par un blocage de la canule.

L'appareil de lipo-aspiration 1 comporte un boîtier 2 qui forme en même temps la poignée de l'appareil. A l'intérieur du boîtier est logé un organe d'entraînement mécanique auquel est reliée la canule 3 d'aspiration, présentant au moins un orifice 14. La canule 3 a de préférence un diamètre de l'ordre de 1,5 à 6 mm, en particulier environ 3, 4 ou 5 mm, en fonction de la couche de graisse à devoir aspirer. Ce diamètre limité permet d'accéder facilement aux endroits du corps humain. La canule a une surface lisse et est de préférence revêtue d'une couche de Teflon ®. Les orifices 14 sont de préférence du nombre de 1 à 3 ayant un diamètre de 2 à 5 mm, les orifices étant de préférence disposés à la hauteur de l'extrémité libre de la canule. Le gaz comprimé est fourni à l'aide d'une conduite 5 reliable à une source de gaz comprimé. La canule 3 est reliée à travers le boîtier à une conduite de décharge 6 qui aboutit dans un réservoir 7 collecteur de la graisse aspirée par la canule. Le réservoir 7 est également relié par une conduite 8 reliée à une unité (non reprise sur le dessin) agencée pour créer une dépression à l'intérieur de la canule, comme par exemple un aspirateur.

Le boîtier 2 comporte également un organe de commande, par exemple un interrupteur 4 qui commande l'arrêt et la mise en marche de l'organe d'entraînement. Selon une autre forme de réalisation, une pédale de commande est prévue pour la mise en marche et l'arrêt de l'appareil. De préférence, l'organe de commande 4 commande également la source d'aspiration qui provoque la dépression à l'intérieur de la canule. Ainsi l'opérateur peut commander par un seul et même mouvement l'ensemble du fonctionnement de l'appareil suivant l'invention.

Suivant une première forme de réalisation l'organe d'entraînement logé à l'intérieur du boîtier comporte un cylindre 10 et un piston pneumatique 11 entraîné par le gaz comprimé fourni par la conduite 5. Le piston 11 agit sur la canule 3 qui est reliée au piston 11. Un capot de protection 13 protège la sortie de la canule à hauteur de la poignée. De préférence, la pression du gaz comprimé est réglée entre 1 à 5 bar, en fonction de la dureté de la graisse à aspirer. Pour une graisse plus dure ou plus fibreuse, on aura tendance à utiliser une pression de 4 à 5 bar, tandis que pour une graisse plus souple et moins fibreuse, on aura tendance à choisir une pression inférieure à 4 bar.

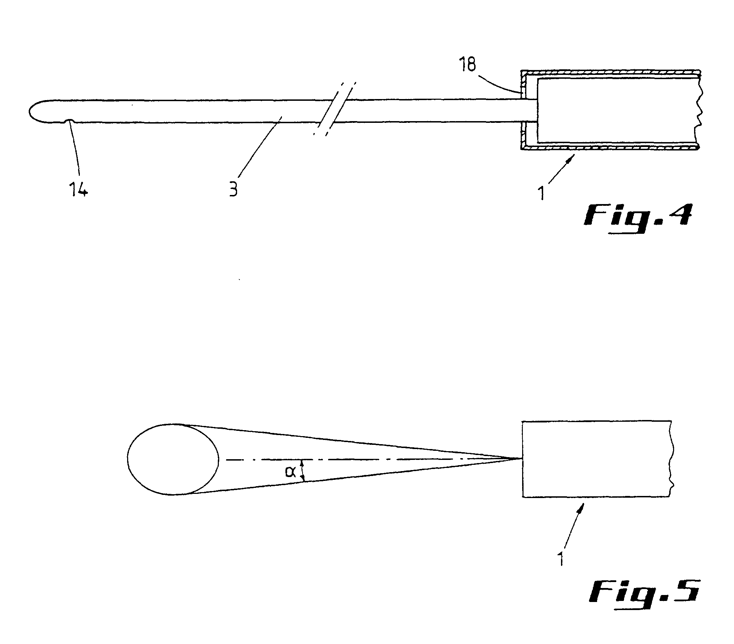

De préférence, comme illustré à la figure 3, la poignée présente une cavité 15 agencée à y placer le pouce de l'opérateur, ladite cavité 15 et ledit orifice 14 étant positionnés de part et d'autre de l'axe 16 de la canule. Ceci provoque que, lorsque l'opérateur, qui a introduit la canule sous la peau 17 d'un patient, place son pouce dans la cavité, l'orifice d'entrée soit dirigé vers la profondeur du tissu adipeux et non vers la peau du patient, assurant ainsi le maintien de l'orifice dans la graisse à aspirer.

Le gaz comprimé fourni au cylindre 10 va imposer un mouvement de va-et-vient au piston 11 à l'intérieur du cylindre. De préférence, le mouvement du piston est imposé par le gaz comprimé dans les deux sens. Il est également concevable que le mouvement du piston soit imposé par le gaz comprimé dans un sens seulement, et que le mouvement dans l'autre sens soit assuré par un ressort. Le mouvement de va-et-vient est produit de préférence à une fréquence située entre environ 10 à 500 Hz, en particulier environ 15 ou 200 Hz. La fréquence utilisée est choisie en fonction du matériau utilisé pour le piston. En effet, les caractéristiques de résonance du matériau choisi influencera l'onde vibratoire. Les matériaux choisis sont par exemple du céramique ou du métal comportant par exemple de l'acier inoxydable ou de l'aluminium. Pour de l'acier inoxydable, on utilise par exemple une fréquence d'environ 15 Hz.

La fréquence de va-et-vient du piston provoque une onde vibratoire au bout de la canule. Un mouvement de nutation est ainsi créé, comprenant un composant de vibration perpendiculaire à l'axe de la canule et un composant de translation suivant l'axe de la canule. L'amplitude du composant de translation est entre 2 mm et 1 cm pour limiter au maximum les lésions des vaisseaux et des nerfs du patient. En particulier l'amplitude est de l'ordre de 3 à 5 mm. Il faut ici noter que des amplitudes de 2 et 6 mm sont donc également comprises.

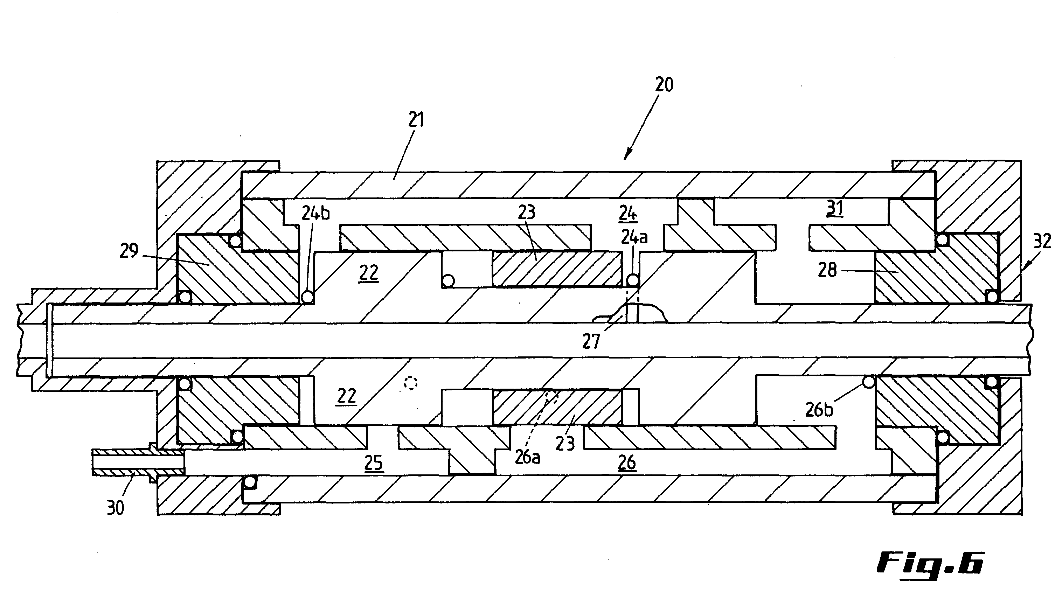

L'amplitude du composant de vibration au bout de la canule est en fonction de la longueur de la canule. Au plus long la canule, au plus grande est l'amplitude du composant de vibration au bout de la canule. La longueur des canules utilisées est de l'ordre de 5 à 35 cm. En utilisant une canule d'une longueur d'environ 25 cm et une fréquence de va-et-vient de 15 Hz, on peut obtenir une amplitude de l'ordre de 1 cm à l'extrémité libre de la canule. Ceci signifie que l'extrémité libre de la canule décrit un mouvement de nutation à l'intérieur d'un cercle, tel qu'illustré à la figure 5, dans laquelle le cercle a un diamètre de l'ordre de 2 cm. La grandeur du mouvement de nutation peut également être exprimée sous forme d'un angle de nutation α tel qu'illustré à la figure 5. Dans le cas d'une canule de 25 cm et une amplitude de 1 cm, l'angle α est de l'ordre de 2,3°. L'amplitude peut également avoir une amplitude inférieure à 1 cm par exemple de l'ordre de 3 à 5 mm.

Pour permettre le mouvement de nutation, un espace libre 18 est prévu entre la canule et la poignée, tel qu'illustré à la figure 4 qui montre en coupe une partie de l'appareil suivant la figure 3.

Ainsi la canule 3 va subir le mouvement de translation qui lui est imposé non seulement lors de l'introduction de la canule sous la peau du patient mais également lors de la lipo-aspiration même. Lors de son utilisation, la vibration de la canule est transmise directement à la graisse ce qui la fait dissocier, disloquer ou éclater et la bat pour ainsi dire en mousse ce qui la fait entrer facilement dans les orifices 14 de la canule, en ne provoquant pratiquement pas de lésions des vaisseaux et des nerfs du patient. La translation permet de faire pénétrer facilement la canule sous la peau, car le mouvement de pénétration, appliqué par l'opérateur, est assisté par le va-et-vient de la canule. En limitant considérablement la lésion, l'appareil peut être même utilisé sous anesthésie locale, ce qui n'est pas le cas dans les appareils à assistance mécanique connus.

L'opérateur ne doit pas impérativement appliquer un massage intensif ni de pinçage de la partie à traiter, comme dans les appareils sans assistance mécanique, car c'est le mouvement de vibration de la canule qui est transmis à la graisse et qui provoque sa dislocation et donc son dégagement vers l'orifice d'entrée de la canule. En pratique, il suffit que l'opérateur tende la peau ou appuie sur la zone à traiter afin de comprimer la graisse.

Puisque l'opérateur ne doit pas impérativement appliquer de massage intensif ni de pinçage il peut se consacrer au guidage de la canule vers les endroits où la graisse doit être disloquée et prélevée. Ceci permet donc à l'opérateur de guider convenablement et avec plus de précision la canule sous la peau du patient. La main de l'opérateur qui tient la poignée peut librement travailler dans toutes les directions de l'espace et les imposer ainsi à la canule. De plus, le mouvement de la canule, puisqu'il est assisté mécaniquement, est plus précis et plus régulier.

Par rapport aux appareils à assistance mécanique connus, l'appareil selon l'invention présente l'avantage qu'il ne produit pas d'effet de guillotine, douloureux pour le patient et provoqué par le mouvement de translation de la canule intérieure par rapport à la canule extérieure.

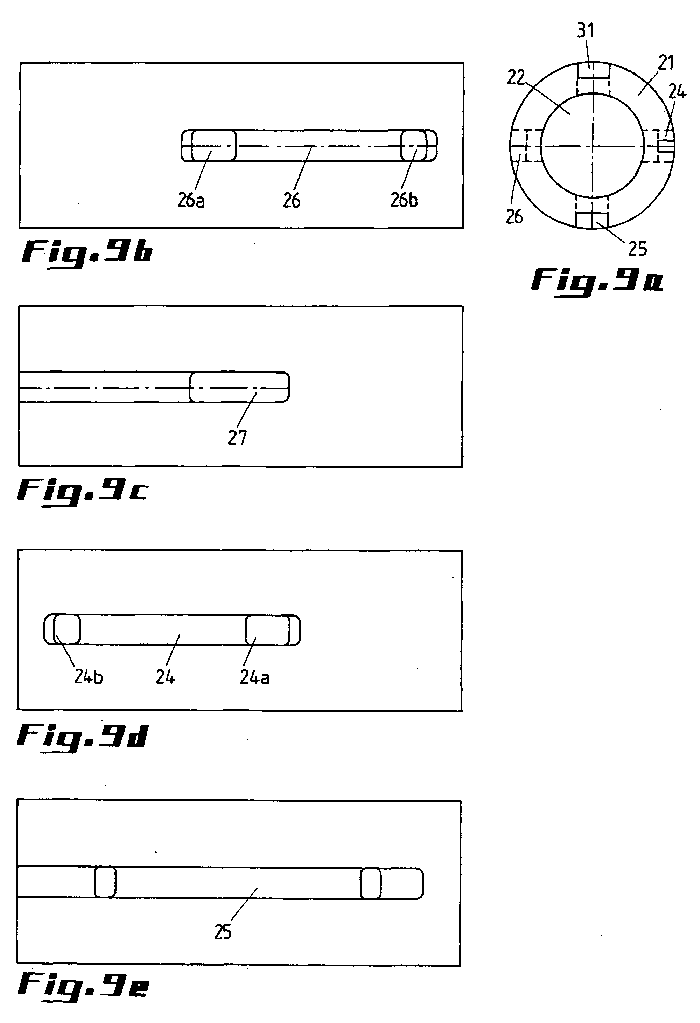

Les figures 6 à 8 illustrent une vue en coupe longitudinale d'une deuxième forme de réalisation de l'organe d'entraînement. Cet organe 20 est également logé dans un boîtier 21 et comporte un piston 22 ainsi qu'un circuit d'admission et d'échappement illustré plus en détail à la figure 9. L'organe comporte un circuit d'admission de gaz, de préférence de l'air comprimé, avant 26 et arrière 24 ayant chacun un injecteur avant (24a, 26a) et arrière (24b, 26b) permettant d'injecter le gaz dans le circuit. Le gaz est fourni par un canal d'admission 27 en liaison avec les injecteurs. L'échappement du gaz est assuré par un circuit d'échappement ayant une partie avant 25 et arrière 31. Dans les figures 6 à 8 les circuits d'échappement sont ramenés dans le plan du dessin alors qu'en réalité ils forment un angle de 90° avec les circuits d'admission. Ceci a été fait pour rendre la description plus facile à comprendre et mieux expliquer le fonctionnement. La figure 9 a illustre une vue en coupe transversale alors que les figures 9 b à e illustrent chaque fois des coupes longitudinales à la hauteur où sont situés les circuits d'admission et d'échappement. Un sélecteur 23 est logé dans un évidement de la paroi extérieure du piston 22. Le déplacement du piston 22 à l'intérieur de l'organe d'entraînement est guidé à l'aide des paliers 28 et 29. Les circuits d'échappement 25 et 31 sont en liaison avec une tubulure 30 permettant le dégagement des gaz d'échappement.

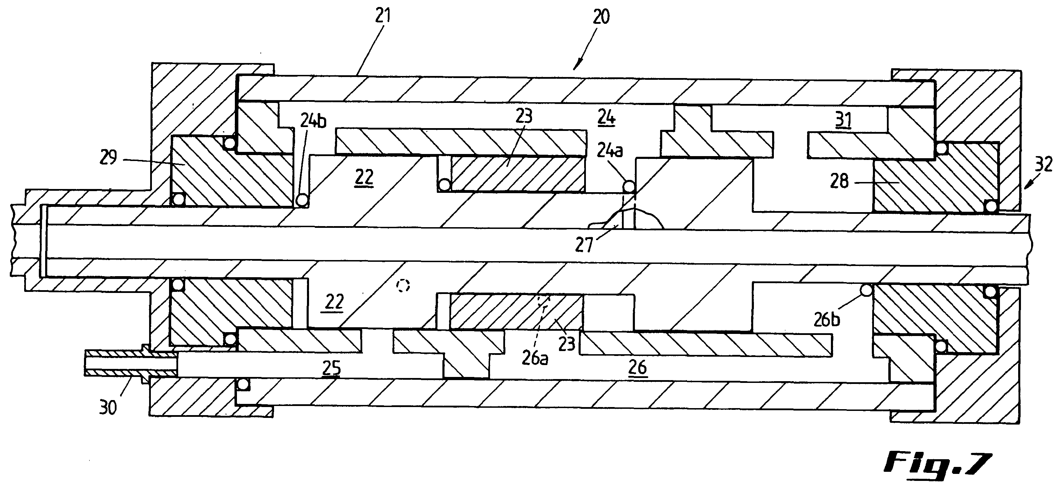

Lorsque le gaz comprimé est fourni au canal d'admission et que le piston 22 est en position initiale arrière, comme illustré à la figure 6, le gaz pénétrera par l'injecteur avant 24a dans le circuit d'admission arrière 24. Puisque l'injecteur avant 24a se trouve près du sélecteur 23, le gaz introduit va exercer une pression sur le sélecteur 23 provoquant ainsi un déplacement de ce dernier vers la gauche ou vers l'arrière de l'organe. Le déplacement vers l'arrière du sélecteur 23 ouvrira d'avantage l'admission vers le circuit d'admission 24 comme illustré à la figure 7 où le sélecteur se trouve en position arrière.

Le circuit d'admission 24 peut maintenant rapidement se remplir de gaz comprimé qui de ce fait cheminera vers l'arrière du circuit d'admission 24. Le déplacement du sélecteur a également ouvert l'injecteur arrière 24b provoquant ainsi également un apport de gaz comprimé à l'arrière du piston 22. La force exercée par le gaz sur l'arrière du piston va maintenant provoquer son déplacement vers la droite ou l'avant de l'organe d'entraînement. Le déplacement du piston provoque à son tour que le gaz présent à l'avant du piston sera poussé dans le circuit d'échappement 31.

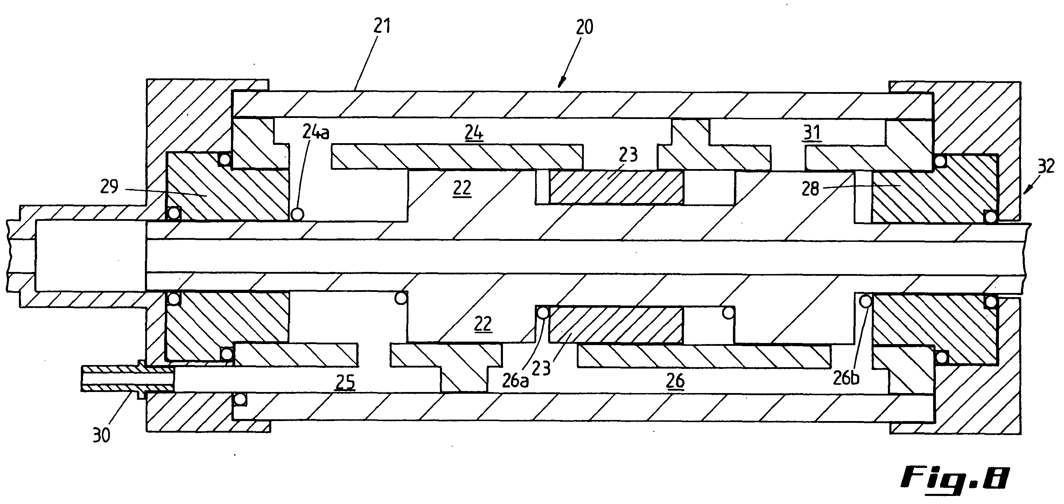

Lorsque le piston 22 a parcouru une course suffisante, il ferme l'admission vers le circuit d'échappement 31 comme illustré à la figure 8. Le gaz qui reste alors dans l'espace entre le palier 28 et le piston 22 va être comprimé par le mouvement du piston et amortit ainsi la course du piston. On obtient ainsi un amortisseur pneumatique qui empêche que le piston vienne percuter le palier 28.

Le mouvement vers l'avant du piston 22 va également comprimer le gaz présent dans le circuit d'admission avant 26, par l'intermédiaire de l'injecteur avant 26b. Le gaz va ainsi circuler dans le circuit d'admission avant 26 pour atteindre l'arrière du circuit. Arrivé à cette position d'équilibre, l'ensemble se trouve alors dans une configuration qui lui permettra de repartir dans le sens opposé.

Puisque le gaz comprimé continu à arriver dans le canal d'admission 27 et que l'injecteur 26a est libre, le gaz peut entrer dans le circuit d'admission avant 26 et exercer une pression sur le sélecteur 23 le poussant ainsi vers l'avant. Le mouvement vers l'avant du sélecteur va ouvrir d'avantage le circuit d'admission avant 26 et l'injecteur 26a, permettant au gaz de cheminer en direction de l'injecteur 26b et de remplir le circuit d'admission avant. Le gaz présent maintenant à l'avant du piston 22 va exercer une pression sur ce dernier et le repousser vers l'arrière. Ce mouvement du piston va provoquer à son tour que le gaz présent dans l'espace entre le palier 29 et le piston sera repoussé dans le circuit 24 et dans le circuit d'échappement 25 d'où il atteint la tubulure 30.

Lorsque le piston 22 a parcouru une course suffisante, il fermera le circuit d'échappement 25 et comprime le gaz dans l'espace entre le palier 29 et le piston. Ainsi le mouvement vers l'arrière du piston est amorti et le gaz dans ledit espace fait fonction d'amortisseur qui empêche que le piston vienne percuter le palier 29. Le gaz dans le circuit d'admission arrière 24 est comprimé et l'on retrouve la situation de départ (figure 6) permettant ainsi de recommencer le mouvement.

La localisation judicieuse des injecteurs d'échappement a pour effet de produire un amortisseur de fin de course, qui limite le déplacement du piston et l'empêche de percuter les paliers. Cette fonction est également exploitée en tant que surpression et permet d'augmenter la compression du gaz dans le circuit d'admission opposé au circuit d'échappement (31 à 24; 25 à 26). Ceci réduit le temps de remplissage du circuit d'admission pour la phase suivante et permet ainsi d'obtenir une fréquence de fonctionnement plus élevée et une économie de la consommation de gaz.

Le mouvement du piston est donc précédé d'un mouvement du sélecteur qui fonctionne en opposition de phase au sens de déplacement du piston.

Le mouvement de va-et-vient qu'exerce le piston 22 et la façon dont le gaz comprimé est alimenté au piston va provoquer le mouvement de nutation de la canule. L'alimentation par petits coups de gaz comprimé provoquant le mouvement de va-et-vient du piston entraîne également un mouvement de flexion sur l'axe du piston. Ces deux mouvements résultent alors dans un mouvement de nutation, qui est permis par un espace libre entre la canule et le boîtier. Le piston a également tendance à exercer un mouvement de rotation qui est toutefois empêché par la présence d'un nez aval 32 à l'avant du piston.

Selon la forme de réalisation illustrée à la figure 1, la graisse, recueillie dans la canule, est collectée latéralement par le tuyau 6 dans le réservoir 7. Selon une alternative, la graisse est collectée par l'intermédiaire de l'axe du piston 11, qui est dans ce cas creux. Ceci facilite considérablement l'évacuation de la graisse, étant donné qu'elle se produit dans la prolongation de la canule.

## Revendications

1. Appareil de lipo-aspiration comprenant une canule d'aspiration (3) agencée pour aspirer de la graisse sous-cutanée par un orifice (14) d'entrée, la canule ayant un axe longitudinal (16) et étant montée sur un organe d'entraînement mécanique (10, 11) logé dans un boîtier (2) ledit organe d'entraînement ayant une entrée pourvue pour y connecter une source d'énergie et agencée pour produire un mouvement et le transmettre à ladite canule et le mouvement de la canule comprenant un composant de translation suivant l'axe de la canule, caractérisé en ce que le mouvement de la canule est un mouvement de nutation comprenant un composant de vibration perpendiculaire à l'axe de la canule et le composant de translation suivant l'axe de la canule, le composant de translation ayant une amplitude entre 2 mm et 1 cm, et un espace (18) étant prévu entre la canule (3) et le boîtier (2), ledit espace étant suffisamment dimensionné de façon à permettre au composant de vibration de provoquer la dislocation de la graisse.

2. Appareil de lipo-aspiration suivant la revendication 1, dans lequel ledit mouvement produit par l'organe d'entraînement a une fréquence entre 10 et 500 Hz, en particulier inférieure à 250 Hz, et plus particulièrement environ égale à 15 Hz.

3. Appareil de lipo-aspiration suivant la revendication 1 ou 2, dans lequel le boîtier (2) forme une poignée.

4. Appareil de lipo-aspiration suivant l'une des revendications précédentes, comportant en outre un organe de commande (4) agencé à commander la mise en marche et l'arrêt de l'organe d'entraînement (10, 11).

5. Appareil de lipo-aspiration suivant la revendication 4 en combinaison avec 3, dans lequel l'organe de commande (4) est un interrupteur, logé dans ladite poignée (2).

6. Appareil de lipo-aspiration suivant la revendication 4, dans lequel l'organe de commande est formé par une pédale de commande coopérant avec ledit organe d'entraînement

7. Appareil de lipo-aspiration suivant l'une des revendications précédentes, dans lequel l'organe d'entraînement est agencé pour fonctionner à l'air comprimé et comporte un piston pneumatique (11) pour produire un mouvement de va-et-vient.

8. Appareil de lipo-aspiration suivant la revendication 7, dans lequel ledit piston (11) comporte un axe creux relié à ladite canule et agencé à évacuer ladite graisse.

9. Appareil de lipo-aspiration suivant l'une des revendications précédentes, présentant une cavité (15) pour un pouce, ladite cavité et ledit orifice étant positionnés de part et d'autre de l'axe (16) de la canule.

## Claims

1. Liposuction device comprising a sucking cannula (3) provided for sucking subcutaneous fat through an entry aperture (14), the cannula having a longitudinal axis (16) and being mounted on a mechanical drive member (10, 11) lodged in a housing (2) said mechanical drive member having an entry provided for connecting an energy source and provided for producing and transmitting a movement to said cannula, and the movement of the cannula comprising a translation component according to the axis of the cannula, characterized in that the movement of the cannula is a nutation movement comprising a vibration component perpendicular to the axis of the cannula and the translation component according to the axis of the cannula, the translation component having an amplitude between 2 mm and 1 cm. and a space (18) being provided between the cannula (3) and the housing (2) said space being sufficiently dimensioned to allow the vibration component to achieve the dislocation of fat.

2. Liposuction device according to claim 1, in which said movement produced by the drive member has a frequency between 10 and 500 Hz, preferably less than 250 Hz, and most preferably approximately equal to 15 Hz.

3. Liposuction device according to claim 1 or 2, wherein the housing (2) of the drive member (10, 11) forms a handle.

4. Liposuction device according to any one of the preceding claims additionally comprising a control member (4) provided for controlling starting and stopping the drive member (10, 11).

5. Liposuction device according to claim 4 in combination with claim 3, wherein the control member (4) is a switch, housed in said handle (2).

6. Liposuction device according to claim 4, wherein the control member is formed by a control pedal cooperating with said drive member.

7. Liposuction according to any one of the preceding claims, wherein the drive member is provided for functioning with compressed air and comprises a pneumatic piston (11) for producing a back and forth motion.

8. Liposuction device according to claim 7, wherein said piston (11) comprises a hollow axis connected to said cannula and provided for evacuating said fat.

9. Liposuction device according to any one of the preceding claims, presenting a cavity (15) for a thumb, said cavity and said aperture being positioned at either side of the axis (16) of the cannula.

## Patentansprüche

1. Fettabsaugvorrichtung, umfassend eine Saugkanüle (3) zum Absaugen von subkutanem Fett durch eine Eingangsöffnung (14), wobei die Kanüle eine Längsachse (16) aufweist und mit einem mechanischen Antriebselement (10, 11) verbunden ist, das sich in einem Gehäuse (2) befindet, wobei das Antriebselement eine Öffnung aufweist, die dazu vorgesehen ist, dort eine Energiequelle anzuschließen und geeignet ist, eine Bewegung hervorzurufen und diese an die Kanüle zu übertragen, und wobei die Bewegung der Kanüle eine Parallelverschiebung entlang der Achse der Kanüle umfasst, dadurch gekennzeichnet, dass die Bewegung der Kanüle unrund ist, und eine zur Kanülenachse senkrechte Vibration umfasst, und die Parallelverschiebung entlang der Kanülenachse verläuft, wobei die Parallelverschiebung eine Amplitude zwischen 2 mm und 1 cm aufweist, und wobei ein Zwischenraum (18) zwischen der Kanüle (3) und dem Gehäuse (2) vorgesehen ist, wobei der Zwischenraum groß genug ist, dass die Vibration die Ablösung des Fetts auslösen kann.

2. Fettabsaugvorrichtung nach Anspruch 1, wobei die Bewegung, die vom Antriebselement hervorgerufen wird, eine Frequenz von zwischen 10 und 500 Hz, bevorzugt von weniger als 250 Hz, besonders bevorzugt von ungefähr 15 Hz aufweist.

3. Fettabsaugvorrichtung nach Anspruch 1 oder 2, wobei das Gehäuse (2) einen Griff bildet.

4. Fettabsaugvorrichtung nach einem der vorhergehenden Ansprüche, die außerdem ein Steuerungselement (4) aufweist, das das Ein- und Ausschalten des Antriebselements (10, 11) steuert.

5. Fettabsaugvorrichtung nach Anspruch 4 in Kombination mit Anspruch 3, wobei das Steuerungselement (4) ein Schalter ist, der sich im Griff (2) befindet.

6. Fettabsaugvorrichtung nach Anspruch 4, wobei das Steuerungselement ein Steuerpedal ist, das mit dem Antriebselement zusammenwirkt.

7. Fettabsaugvorrichtung nach einem der vorhergehenden Ansprüche, wobei das Antriebselement mittels Druckluft angetrieben wird und einen Drucktuftkolben (11) aufweist, der eine Vor- und Zurückbewegung erzeugt.

8. Fettabsaugvorrichtung nach Anspruch 7, wobei der Kolben (11) eine Hohlachse aufweist, die mit der Kanüle verbunden ist und geeignet ist, das Fett zu entfernen.

9. Fettabsaugvorrichtung nach einem der vorhergehenden Ansprüche, die eine Vertiefung (15) für einen Finger aufweist, wobei die Vertiefung und die Öffnung sich auf gegenüberliegenden Seiten der Achse (16) der Kanüle befinden.
